# EUROPEAN PATENT APPLICATION

(11) **EP 4 458 381 A1**
(43) Date of publication of application: **06.11.2024**
(21) Application number: 22916331.6
(22) Date of filing: 17.10.2022
(51) Int. Cl.: A61L 27/56, A61L 27/18, A61L 27/34, A61L 27/36, A61L 27/58

(54) **BIOCOMPATIBLE THREE-DIMENSIONAL SCAFFOLD FOR TISSUE RESTORATION**

(30) Priority: 30.12.2021 KR 20210193211
(71) Applicant: Plcoskin Co., Ltd., Seoul 03721 (KR); Industry-Academic Cooperation Foundation, Yonsei University, Seoul 03722 (KR)
(72) Inventor: BAEK, Wooyeol, Seoul 04036 (KR); LEE, Won Jai, Seoul 04765 (KR); KIM, Dohyun, Seoul 07679 (KR); PARK, Yongkyu, Seoul 02829 (KR); JO, Han Saem, Seoul 08521 (KR); SHIM, Kyu-Sik, Seoul 06677 (KR); HEO, Min, Seoul 04983 (KR)
(74) Representative: Latscha Schöllhorn Partner AG
(86) International application number: PCT/KR2022/015711
(87) International publication number: WO 2023/128177

(57) **Abstract**

The present invention provides a biocompatible three-dimensional scaffold and a composition for reconstructing lost tissue comprising the same. The scaffold of the present invention provides a mechanical support for a tissue cavity due to its strong physical strength and, at the same time, efficiently induces quantitative restoration of irreversibly lost tissue because the internal space thereof may be filled with active ingredients for tissue regeneration. In addition, when the spherical scaffold of the present invention is used, it may be efficiently inserted into a tissue cavity having any shape, and may also be degraded at an appropriate time after the completion of regeneration, indicating that it is useful as a scaffold for human transplantation for restoring various adipose tissues, including breast tissue.

## Description

### Technical Field

The present invention relates to a biocompatible scaffold having a spherical or near-spherical geometry and a method of reconstructing breast tissue after lumpectomy or the like using the same.

### Background Art

Breast cancer refers to a malignant tumor that develops from cells in the breast. Although the cause of breast cancer has not been clearly identified, various factors such as female hormones, family history, past history, childbirth history, and dietary habits have been mentioned as the cause. The 5-year survival rate of breast cancer is known to be less than 20% for stage 4 breast cancer, compared to 100% for stage 0 breast cancer. In women who are experiencing physiologically active physical changes such as low birth rate, short lactation period, early menarche, and late menopause, the incidence of breast cancer has recently been increasing rapidly due to the increased sensitivity of mammary gland tissue caused by a rapid increase in the number of stimulations by female hormones, the westernization of diet, pollution of the living environment, etc. This increase in the incidence of breast cancer and the increase in the mortality rate due to breast cancer are expected to continue for a considerable period of time considering the current trend of westernization.

As of 2020, more than 2 million people have been newly diagnosed with breast cancer in Korea every year, and the prevalence rate of breast cancer has increased by 5% every year. Among surgical operations for early-stage breast cancer, partial mastectomy (lumpectomy) has gradually increased, and as of 2018, it is twice the rate of total mastectomy. In reconstruction after lumpectomy, the methods of augmenting the breast contralateral to the resected breast, or inserting an implant into the resected breast, or transplanting autologous fat collected from the abdomen, etc. into the resected breast are mainly used. Contralateral breast augmentation has disadvantages in that it is expensive and new scars are formed in areas other than the lesion, even though it is a cosmetic surgery unrelated to survival. In the method of inserting an implant to correct the appearance, the problem of high cost arises due to the use of expensive cadaver-derived dermis, and the reconstruction effect is also unsatisfactory. Meanwhile, in the case of autologous fat transplantation, there is a risk of skin-soft tissue fibrosis and adhesion due to radiation treatment, and there is a disadvantage that the effect of plastic surgery disappears as the fat tissue is absorbed over time after transplantation. Accordingly, there is a need for the development of a new therapeutic approach that efficiently and safely reconstructs lost tissue.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made extensive research efforts to develop a three-dimensional scaffold for tissue regeneration that efficiently restores a tissue defect caused by trauma or surgical resection. As a result, the present inventors have found that, when a spherical or hemispherical, specifically spherical porous scaffold, which is composed of biocompatible polymer and is hollow inside, is inserted into a tissue cavity, it may not only provide a mechanical support for the tissue cavity due to its strong physical strength and, at the same time, induce quantitative reconstruction of lost tissue because the internal space thereof may be loaded with active ingredients that help tissue regeneration, but also be degraded at an appropriate time after its insertion, indicating that it is useful as a scaffold for human transplantation for reconstruction of breast tissue, etc., thereby completing the present invention.

Therefore, an object of the present invention is to provide a three-dimensional scaffold for tissue regeneration.

Another object of the present invention is to provide a composition for reconstructing lost tissue.

Other objects and advantages of the present invention will be more apparent from the following detailed description, the appended claims and the accompanying drawings.

### Technical Solution

According to one aspect of the present invention, the present invention provides a three-dimensional scaffold for tissue regeneration comprising:
(a) at least one spherical or hemispherical porous layer composed of a biocompatible polymer; and
(b) a cavity formed inside the porous layer.

The present inventors have made extensive research efforts to develop a three-dimensional scaffold for tissue regeneration that facilitates efficient reconstruction of irreversibly lost tissue and, in particular, restores a tissue defect caused by trauma or surgical resection. As a result, the present inventors have found that, when a spherical or hemispherical, specifically spherical porous scaffold, which is composed of a biocompatible polymer and is hollow inside, is inserted into a tissue cavity, it may not only provide a mechanical support for the tissue cavity due to its strong physical strength and, at the same time, induce quantitative reconstruction of lost tissue because the internal empty space thereof may be loaded with active ingredients that help tissue regeneration, but also be degraded at an appropriate time after its insertion, indicating that it is useful as a scaffold for human transplantation for restoring various tissues, including breast tissue.

In the present specification, the term "structure" or "scaffold" refers to a tissue engineering scaffold serving to promote the repair and regeneration of damaged tissue by mounting living cells, specifically cells derived from damaged tissue or cells involved in the repair of damaged tissue. Specifically, the scaffold of the present invention may be either a single-layer scaffold composed of a single porous sphere or a core-shell multi-layer scaffold in which a plurality of spheres are stacked.

In the present specification, the term "spherical" refers to a three-dimensional shape composed of a set of points having the same distance from the center, but the "spherical" porous layer of the present invention does not have to be a geometrically perfect sphere, and includes any structure in which the distance from each point on the strand constituting the porous layer to the center does not deviate from a certain range.

According to a specific embodiment of the present invention, the spherical or hemispherical porous layer of the present invention has a geometric structure selected from the group consisting of an icosahedron, a truncated icosahedron, and a truncated octahedron.

According to another embodiment of the present invention, the spherical or hemispherical porous layer of the present invention may have a spherical or near-spherical shape formed by three-dimensionally molding a planar radial structure, and in this case, it has a curved surface, not a polyhedral surface.

Most specifically, the spherical or hemispherical porous layer of the present invention has the geometry of a truncated icosahedron. In the present specification, the term "truncated icosahedron" refers to a soccer ball-shaped polyhedron made when truncating each vertex (trisection of edges) of an icosahedron, each face of which is an equilateral triangle.

When a scaffold having a spherical or near-spherical geometry as described above is used, it may be efficiently inserted into and fully fill a tissue cavity regardless of the shape of the tissue cavity. In addition, the scaffold may be standardized and produced regardless of the shape or volume of the lost tissue to be restored.

According to a specific embodiment of the present invention, the porous layer may be formed by intersecting biocompatible polymer strands, and may be composed of a combination of two or more biocompatible polymers.

According to the present invention, the spherical (or hemispherical) porous layer of the present invention may be formed by intersecting strands having a certain thickness at regular intervals to form pores of a certain size.

According to a specific embodiment of the present invention, the strand has a diameter of 0.1 to 50 mm. If the diameter of the strand is smaller than 0.1 mm, it is difficult to maintain the shape of the three-dimensional scaffold after insertion into the human body, which reduces the scaffold role. If the diameter is larger than 50 mm, tissue regeneration is difficult, biodegradation takes a long time, and elasticity is reduced. More specifically, the strand has a diameter of 0.5 to 30 mm, even more specifically a diameter of 0.7 to 20 mm, even more specifically a diameter of 0.8 to 1.5 mm, most specifically about 1 mm.

In the present specification, the term "polymer" refers to a synthetic or natural polymer compound in which the same or different types of monomers are sequentially bonded. Accordingly, polymers include homopolymers (polymers produced by polymerization of one type of monomer) and interpolymers produced by polymerization of at least two different monomers, wherein the interpolymers include copolymers (polymers produced from two different monomers) and polymers produced from more than two different monomers.

In the present specification, the term "biocompatibility" refers to the property of not causing short-term or long-term side effects when administered *in vivo* and coming into contact with organ cells, tissues, or body fluids. Specifically, the term "biocompatibility" is meant to include not only tissue compatibility and blood compatibility meaning that the scaffold does not cause tissue necrosis or blood coagulation in contact with biological tissue or blood, but also biodegradability meaning that the scaffold is biodegraded after a certain period of time after administration *in vivo.*

In the present specification, the term "biodegradability" refers to the property of being naturally degraded when exposed to a physiological solution of pH 6-8. Specifically, the term "biodegradability" refers to the property of being degradable over time by body fluids, biodegradation enzymes, or microorganisms *in vivo.*

Biocompatible polymers that may be used in the present invention include, but are not limited to, poly(caprolactone) (PCL), poly(2-hydroxyethyl methacrylate) (HEMA), polyvinyl alcohol (PVA), polyethylene oxide (PEO), phospholipid, collagen, aliphatic polyether, poly(lactide) (PLA), poly(glycolide) (PGA), poly(dioxanone) (PDO), poly(butyrolactone) (PBL), poly(valerolactone) (PVL), poly(lactide-co-glycolide) (PLGA), polyurethane (PU), fibronectin, vitronectin, poly(L-lysin), poly(L-glutamic acid), poly(aspartic acid), carboxymethyl cellulose, cellulose sulfate, agarose, alginate, carrangeenan, hyaluronic acid, dextran, chitosan, poly(hydroxybutyric acid), poly(alkylene succinate), polyamide, poly(anhydride), poly(ortho-ester), poly(cyanoacrylates), polyphosphazene, poly(hydroxyethyl methacrylate), poly(methyl methacrylate), poly(tetrafluoroethylene), poly(dimethylsiloxane), poly(ethylene oxide-β-propylene oxide), poly(vinylmethylether), poly(N-alkylacrylamide), decellularized matrix (dECM), and combinations thereof. More specifically, the biocompatible polymer that is used in the present invention is aliphatic polyester, poly(lactide) (PLA), poly(glycolide) (PGA), poly(dioxanone) (PDO), poly(butyrolactone) (PBL), poly(valerolactone) (PVL), poly(lactide-co-glycolide) (PLGA), poly(caprolactone) (PCL), or a combination thereof. Most specifically, the biocompatible polymer is poly(caprolactone) (PCL).

The biocompatible three-dimensional scaffold of the present invention may be produced by coating biocompatible polymer strands with collagen.

In the present specification, the term "coating" means forming a new layer of a certain thickness on a target surface by modification with a specific material, wherein the target surface and the coating material may be modified through ionic or non-covalent bonds. The term "non-covalent bonds" is a concept that includes physical bonds such as adsorption, cohesion, chain entanglement and entrapment, as well as bonds caused by interactions such as hydrogen bonds and van der Waals bonds alone or in combination with the physical bonds described above. In the present invention, when the strands constituting the scaffold are coated with the collagen solution, a closed layer may be formed while completely surrounding the surface of each strand, or a partially closed layer may be formed.

According to a specific embodiment of the present invention, the scaffold has a diameter of 3 to 200 mm. If the diameter of the scaffold is less than 3 mm, significant adipose tissue regeneration is not smooth, and if the scaffold has a diameter of 200 mm or more, its insertion into the tissue cavity is difficult.

More specifically, the scaffold has a diameter of 10 to 100 mm, more specifically 15 to 50 mm, even more specifically 20 to 40 mm, most specifically about 30 mm.

According to another aspect of the present invention, the present invention provides a composition for reconstructing lost tissue, comprising: the above-described scaffold of the present invention; and collagen, adipose tissue, mammary tissue, or a combination thereof as an active ingredient.

As described above, the empty space of the scaffold of the present invention may be loaded with active ingredients that help tissue regeneration, including collagen, adipose tissue, and mammary tissue. The active ingredients for tissue regeneration may be appropriately selected from collagen, adipose tissue, and mammary tissue, as well as cells or tissue lumps derived from the tissue to be regenerated depending on the nature of the tissue. In addition, the active ingredient that helps tissue regeneration may be transplanted into the lesion in a state filled in the internal space of the scaffold of the present invention, or the scaffold may be first transplanted into the lesion and then the internal space of the scaffold may be filled with the active ingredient before suturing.

In the present specification, the term "transplantation" refers to the process of transferring living tissue, cells, active ingredients that help tissue regeneration, or an artificial scaffold containing them from a donor to a recipient with the goal of maintaining the functional integrity of the tissue or cells transplanted into the recipient. Accordingly, the term "scaffold for transplantation" refers to a physical scaffold that is used in the process of transferring living tissue or cells to a recipient.

According to a specific embodiment of the present invention, the lost tissue is adipose tissue or fibrous tissue. More specifically, the adipose tissue is breast adipose tissue.

According to still another aspect of the present invention, the present invention provides a radial porous mesh composed of a biocompatible polymer for forming the above-described three-dimensional scaffold for tissue regeneration according to the present invention. In the porous mesh of the present invention, a plurality of straight meshes extending from the center may form a radial shape in the shape of spokes, and may be rounded into a spherical shape so that their corresponding ends meet, thereby forming the "above-described spherical porous layer having a cavity" of the present invention.

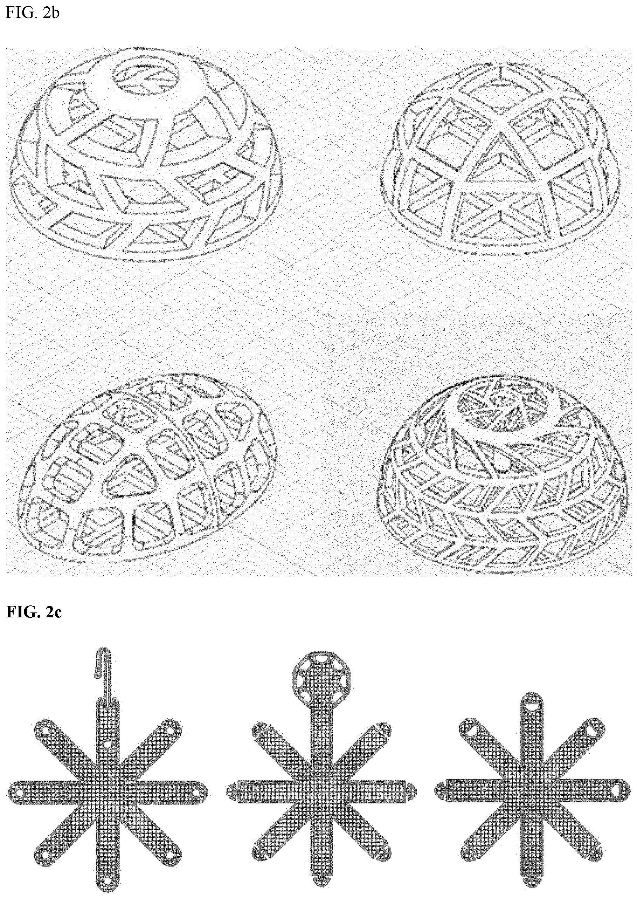

According to a specific embodiment of the present invention, the porous mesh may comprise a fixing portion and a fixing portion-receiving portion at both ends facing each other with respect to the center of the radial shape, respectively. In this way, the corresponding two ends of the straight meshes extending from the center in the shape of spokes may be mechanically coupled through the fixing portion and the fixing portion-receiving portion, respectively, so that the spherical porous layer may become more solid. This fixing portion and fixing portion-receiving portion may be formed only at one pair of corresponding ends (left side of FIG. 2c), or may be formed at multiple pairs of corresponding ends, or may be formed at all pairs of corresponding ends (right side of FIG. 2c).

In addition, when the fixing portion is formed at one end, the fixing portion-receiving portion may be formed not only at the corresponding end, but also at all other ends, so that one fixing portion and a plurality of fixing portion-receiving portions may be coupled (left side of FIG. 2c).

According to another specific embodiment, the porous mesh has n ends extending from a center of the radial shape, and of the n ends, n-1 ends comprise a fixing portion or a fixing portion-receiving portion, and one end comprises n-1 fixing portions or fixing portion-receiving portions which may form couplings with the fixing portions or the fixing portion-receiving portions included in the remaining n-1 ends (middle of FIG. 2c).

For example, the fixing portion and the fixing portion-receiving portion may be a hook and a ring-shaped receiving portion, respectively, but are not limited thereto, and any fastening means capable of forming a mechanical coupling and fixing the formed coupling may also be applied.

According to yet another aspect of the present invention, the present invention provides a method for reconstructing lost tissue in a subject, comprising a step of transplanting, into the cavity of the lost tissue in the subject, the above-described scaffold of the present invention, and collagen, adipose tissue, mammary tissue, or a combination thereof.

Since the scaffold, collagen, adipose tissue, and mammary tissue used in the present invention have already been described in detail, description thereof will be omitted to avoid excessive overlapping.

### Advantageous Effects

The features and advantages of the present invention are summarized as follows:
(a) The present invention provides a biocompatible three-dimensional scaffold and a composition for reconstructing lost tissue comprising the same.
(b) The scaffold of the present invention provides a mechanical support for a tissue cavity due to its strong physical strength and, at the same time, efficiently induces quantitative restoration of irreversibly lost tissue because the internal space thereof may be loaded with active ingredients that help tissue regeneration.
(c) When the spherical scaffold of the present invention is used, it may be efficiently inserted into a tissue cavity having any shape, and may also be degraded at an appropriate time after the completion of regeneration, indicating that it is useful as a scaffold for human transplantation for restoring various adipose tissues, including breast tissue.

### Brief Description of Drawings

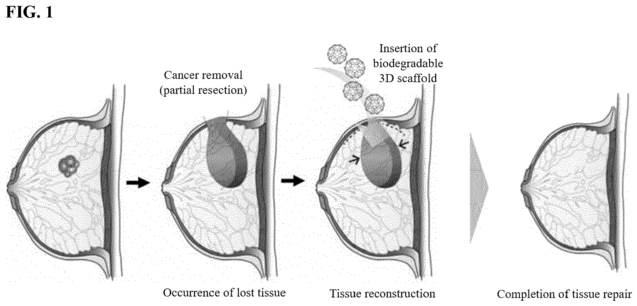

FIG. 1 is a schematic diagram showing the process of partially restoring lost breast tissue by applying the biocompatible three-dimensional structure of the present invention, and shows that a sunken breast may be restored by inserting the scaffold of the present invention into a cavity that occurs after lumpectomy.
FIG. 2 shows the biocompatible three-dimensional scaffold structures of the present invention having various strand shapes, and shows spherical scaffolds (FIG. 2a), hemispherical scaffolds (FIG. 2b), and radial structures for forming spherical scaffolds (FIG. 2c).
FIG. 3 shows micro-CT images obtained for the cross-section (FIG. 3a) and longitudinal section (FIG. 3b) of the transplanted sites 2, 4, and 6 months after transplantation of the biocompatible three-dimensional scaffold of the present invention into rat models, and also shows the results of H&E staining and Masson's trichrome staining after horizontal sectioning of the tissue of the transplanted sites.
FIG. 4 shows the results of performing H&E staining and Masson's trichome staining after sectioning the tissue of the transplanted site 3 months after transplanting the biocompatible three-dimensional scaffold of the present invention into pig models.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Preparation Example 1: Fabrication of Biocompatible Three-Dimensional Scaffolds

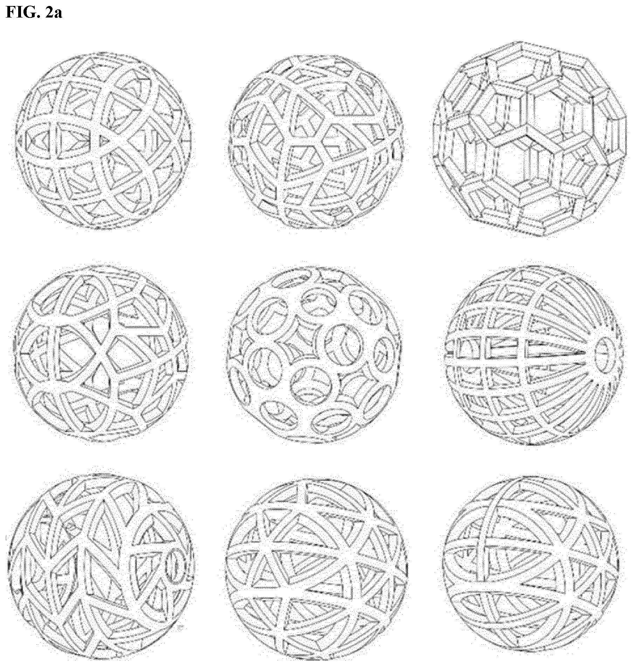

A 3D printer (RAISE 3D, USA) was used to fabricate three-dimensional polymer scaffolds, and the 3D printing technique could easily adjust the size of the three-dimensional scaffolds according to conditions such as the diameter of the nozzle, the nozzle temperature, discharge pressure, and the moving speed of the nozzle. The present inventors fabricated three-dimensional scaffolds with an overall spherical shape by geometrically intersecting strands having a diameter of 1 mm so that each of the scaffolds would have high mechanical elasticity while occupying the volume of the missing portion in tissue. Polycaprolactone (PCL, Sigma-Aldrich, USA) was used as the raw polymer, and to produce the polymer mesh, the nozzle diameter was set to 0.4 mm, the nozzle temperature to 130°C, the output speed to 5 mm/s, and the moving speed of the nozzle to 40 mm/s. After the setting was completed, three-dimensional scaffolds having a truncated icosahedral structure with a diameter of 1 cm and a strand thickness of 1 mm was fabricated. Before use in subsequent experiments, the scaffolds were sterilized with an e-beam. The fabricated three-dimensional polymer scaffolds were hollow spherical scaffolds having meshes of various shapes formed on their surface by intersecting strands in various configurations (FIG. 2).

### Preparation Example 2: Fabrication of Biocompatible Three-Dimensional Scaffolds Coated with Collagen

Collagen coating using a collagen solution was additionally performed on the biocompatible three-dimensional scaffolds fabricated in Preparation Example 1. The collagen solution was prepared by dissolving atellocollagen (type 1, medical device grade, Dalim Tissen Co., Ltd. Korea), extracted from porcine dermis, in 0.5M acetic acid at a concentration of 0.5% at 4°C for 12 hours.

For uniform coating with collagen, highly hydrophobic polycaprolactone was rendered hydrophilic by surface treatment using plasma. The three-dimensional scaffold of polycaprolactone was placed on a glass Petri dish and then treated using a plasma surface treatment machine (PDC-32G-2 Plasma, Harrick Plasma, USA) for 60 seconds under a medium vacuum condition of 1.0-0.1 Torr.

After the surface treatment process, the three-dimensional scaffold was placed on a plate with a certain height, and the collagen solution was added so that the three-dimensional structure could be sufficiently submerged, followed by coating at 4°C for 30 minutes. The coated sample was cooled to -50°C for 3 hours and then dried using a freeze dryer (MG-VFD5, MG Indus., Korea) over 14 hours to make a porous surface structure of collagen coated on the surface.

Thereafter, neutralization was performed to remove acetic acid present as a salt inside the freeze-dried collagen. To this end, the freeze-dried sample was washed four times with absolute alcohol (ethanol absolute, Merck KGaA, Germany) for 15 minutes using, and then neutralization of acetic acid was performed four times with a solution of 0.5 M NaOH (Duksan General Science, Korea) in 70% ethanol for 15 minutes. Next, to remove the remaining amount of NaOH present in the sample, the sample was washed four times sequentially with 50% and 30% ethanol and triple distilled water for 15 minutes. The washed collagen-coated three-dimensional scaffold was cooled to -50°C for 3 hours and then dried using a freeze dryer for 12 hours as mentioned above. Thereafter, the scaffold was sterilized with an e-beam before use in experiments.

The fabricated three-dimensional polymer scaffolds were hollow spherical scaffolds having meshes of various shapes formed on their surface by intersecting strands in various configurations.

### Experimental Example 1: Animal Experiment Using Rat Model

The present inventors evaluated whether the introduction of the three-dimensional polymer scaffold of the present invention could be applied to tissue regeneration, for example, partial breast reconstruction. For experiments, SD rats (8-week-old, female) were divided into a total of four groups: a group in which about 50% of the tissue of the mammary gland was resected with surgical scissors, followed by suturing; a group in which about 50% of the tissue of the mammary gland was excised with surgical scissors, and then the three-dimensional scaffold (PCL ball) of Preparation Example 1 was transplanted into the resected part of the mammary gland, followed by suturing; a group in which about 50% of the tissue of the mammary gland was excised with surgical scissors, and then the three-dimensional scaffold (PCL-col ball) of Preparation Example 2 was transplanted into the resected part of the mammary gland after injecting the collagen preparation into the three-dimensional scaffold, followed by suturing; and a group in which about 50% of the tissue of the mammary gland was resected with surgical scissors, and then the three-dimensional scaffold of Preparation Example 2 was transplanted, and mouse mammary gland tissue was inserted into the three-dimensional scaffold, followed by suturing.

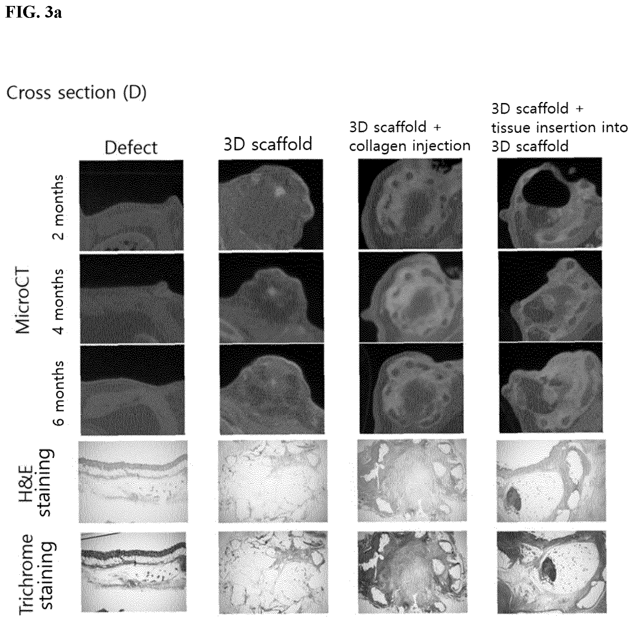

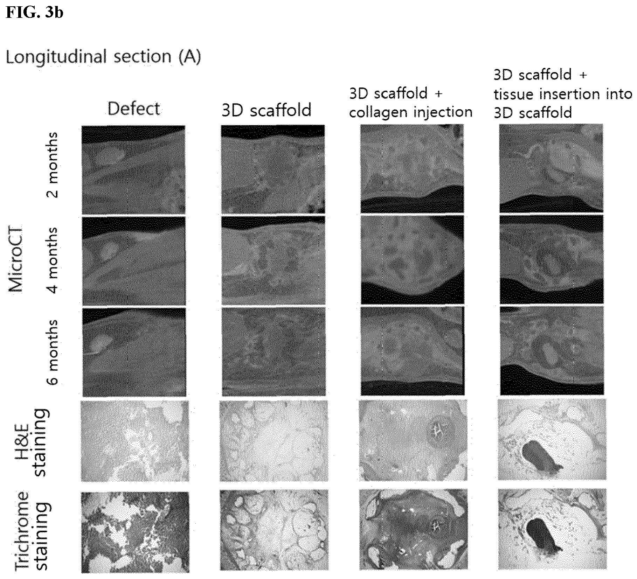

At 2 months, 4 months and 6 months after suturing, the transplanted site was imaged using microCT (Quantum GX2 microCT Imaging System, PerkinElmer), and each experimental animal was sacrificed, and the tissue of the area transplanted with the three-dimensional scaffold was extracted, sectioned in the vertical or horizontal direction, and then subjected to hematoxylin & eosin (H&E) staining and Masson's trichrome staining. As a result, as shown in FIGS. 3a and 3b, the results of microCT imaging at 2 months, 4 months and 6 months for the mice that were not treated with anything after resecting about 50% of the mammary gland tissue indicated that the lost tissue was not restored at all, whereas the results of microCT imaging at 2 months, 4 months and 6 months for the mice transplanted with the three-dimensional scaffold of the present invention indicated that tissue was significantly formed within the three-dimensional scaffold over time. It was confirmed that, in the case of the group in which the collagen preparation was injected into the three-dimensional scaffold after transplantation of the three-dimensional structure, tissue that appeared opaque on the microCT image was continuously maintained within the three-dimensional scaffold. It could be confirmed that, in the case of the group in which the three-dimensional scaffold was filled with the mouse mammary tissue after implantation of the three-dimensional structure, followed by suturing, the tissue gradually grew within the scaffold over time. It could be confirmed through H&E staining that, in all of the cases in which only the three-dimensional scaffold was transplanted, in which the three-dimensional structure injected with the collagen preparation was transplanted, and in which the three dimensional scaffold having the mammary gland tissue injected therein was transplanted, tissue was generated in the internal space (cavity). In particular, it was confirmed that, in the group in which the three-dimensional scaffold was transplanted, adipose tissue was generated around the scaffold and entered the scaffold, and in the three-dimensional scaffold into which the collagen preparation was injected, regeneration of fibrous tissue occurred. In addition, it was observed that, in the three-dimensional scaffold into which the mammary gland tissue was inserted, the mammary gland tissue was well maintained inside the scaffold and fibrous tissue was regenerated while surrounding the mammary gland tissue.

In addition, it was observed through Masson's trichrome staining that more collagen was produced inside the three-dimensional scaffold injected with the collagen preparation compared to the three-dimensional structure. Considering that the injected collagen is usually degraded within 1 to 2 months, it was confirmed that fibrous tissue was formed by the injected collagen, and at 6 months, the tissue was composed and restored by collagen secreted from the fibrous tissue. In addition, it could be seen that that, inside the three-dimensional scaffold into which the mammary gland tissue was inserted, the remaining tissues, excluding the mammary gland tissue, were fibrous tissues composed of collagen.

### Experimental Example 2: Animal Experiment Using Pig Model

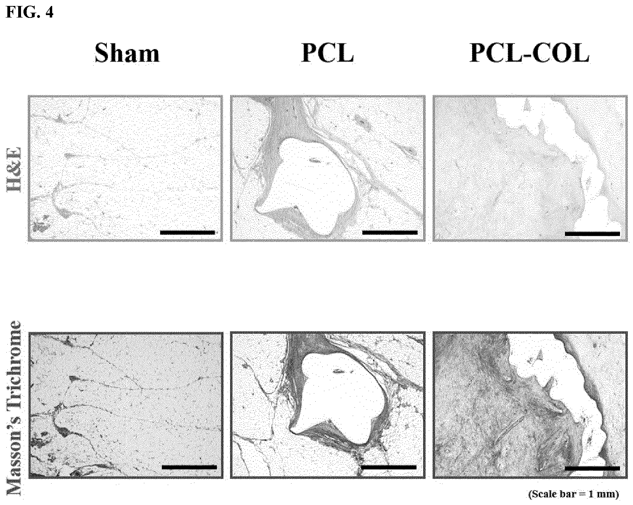

To produce minipig lumpectomy models, 12-month-old adult mini pigs were bred in an SPF breeding facility. After an acclimatization period of 1 week, anesthesia was induced by intramuscular injection of 1 mg/kg alfaxane and 2 mg/kg xylazine, and then anesthesia was maintained by respiratory anesthesia of 2% isoflurane during surgery. After sterilizing the surgical site of the minipig with 70% ethanol and iodine, 15 ml of mammary gland tissue was partially resected to produce partial resection models. The three-dimensional structure of each of Preparation Example 1 (PCL ball) and Preparation Example 2 (PCL-col ball) was transplanted into the partially resected surgical area, followed by suturing. Next, 20 mg/kg cefazolin and 0.4 mg/kg meloxicam were injected intramuscularly for 3 days. Three months after the surgery, the minipigs were sacrificed by injecting KCL under general anesthesia, and the transplanted PCL ball and PCL-col ball were collected. Thereafter, the cells and tissue were washed three times with PBS (phosphate-buffered saline) and then fixed by treatment with a 10% formalin solution. Next, hematoxylin & eosin (H&E) staining and Masson's trichrome staining were performed to characterize the fixed tissue. As a result, as shown in FIG. 4, it could be confirmed that, 3 months after transplantation into the lumpectomy model, tissue derived from the inside of the minipig was introduced and generated inside the PCL ball and PCL-col ball. In addition, it was confirmed that, in the group transplanted with the PCL ball, a small amount of fibrous tissue was generated inside the PCL ball and autologous mammary tissue was introduced from the outside. It could be confirmed that, in the group transplanted with PCL-col, a large amount of fibrous tissue was generated inside the PCL-col ball, and that almost no autologous mammary tissue was introduced from the outside.

Although the present disclosure has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A three-dimensional scaffold for tissue regeneration comprising:
(a) at least one spherical or hemispherical porous layer composed of a biocompatible polymer; and
(b) a cavity formed inside the porous layer.

2. The scaffold of claim 1, wherein the porous layer is formed by intersecting biocompatible polymer strands.

3. The scaffold of claim 1, wherein the biocompatible polymer is selected from the group consisting of poly(caprolactone) (PCL), poly(2-hydroxyethyl methacrylate) (HEMA), polyvinyl alcohol (PVA), polyethylene oxide (PEO), phospholipid, collagen, aliphatic polyether, poly(lactide) (PLA), poly(glycolide) (PGA), poly(dioxanone) (PDO), poly(butyrolactone) (PBL), poly(valerolactone) (PVL), poly(lactide-co-glycolide) (PLGA), polyurethane (PU), fibronectin, vitronectin, poly(L-lysin), poly(L-glutamic acid), poly(aspartic acid), carboxymethyl cellulose, cellulose sulfate, agarose, alginate, carrangeenan, hyaluronic acid, dextran, chitosan, poly(hydroxybutyric acid), poly(alkylene succinate), polyamide, poly(anhydride), poly(ortho-ester), poly(cyanoacrylates), polyphosphazene, poly(hydroxyethyl methacrylate), poly(methyl methacrylate), poly(tetrafluoroethylene), poly(dimethylsiloxane), poly(ethylene oxide-β-propylene oxide), poly(vinylmethylether), poly(N-alkylacrylamide), decellularized matrix (dECM), and combinations thereof.

4. The scaffold of claim 3, wherein the biocompatible polymer is poly(caprolactone) (PCL).

5. The scaffold of claim 2, wherein the strands have a diameter of 0.1 to 50 mm.

6. The scaffold of claim 5, wherein the strands are coated with collagen.

7. The scaffold of claim 1, wherein the scaffold has a diameter of 3 to 200 mm.

8. The scaffold of claim 1, wherein the spherical or hemispherical porous layer has a geometric structure selected from the group consisting of an icosahedron, a truncated icosahedron, and a truncated octahedron.

9. The scaffold of claim 1, wherein the spherical or hemispherical porous layer is formed by three-dimensionally molding a planar radial structure.

10. A composition for reconstructing lost tissue, comprising: the scaffold of any one of claims 1 to 9; and collagen, adipose tissue, mammary tissue, or a combination thereof as an active ingredient.

11. The composition of claim 10, wherein the lost tissue is adipose tissue or fibrous tissue.

12. The composition of claim 11, wherein the adipose tissue is breast adipose tissue.

13. A radial porous mesh composed of a biocompatible polymer for forming the three-dimensional scaffold for tissue regeneration according to claim 1.

14. The porous mesh of claim 13, wherein the porous mesh comprises a fixing portion and a fixing portion-receiving portion at both ends facing each other with respect to a center of the radial shape, respectively.

15. The porous mesh of claim 13, wherein the porous mesh has n ends extending from a center of the radial shape, and of the n ends, n-1 ends comprise a fixing portion or a fixing portion-receiving portion, and one end comprises n-1 fixing portions or fixing portion-receiving portions which may form couplings with the fixing portions or the fixing portion-receiving portions included in the remaining n-1 ends.
